(19) Europäisches Patentamt
European Patent Office
Office européen des brevets

(11) EP 2 959 944 A1

(12) **EUROPEAN PATENT APPLICATION**
published in accordance with Art. 153(4) EPC

(43) Date of publication:
**30.12.2015 Bulletin 2015/53**

(51) Int Cl.:
**A61N 5/10** (2006.01)

(21) Application number: **13875865.1**

(86) International application number:
**PCT/JP2013/054568**

(22) Date of filing: **22.02.2013**

(87) International publication number:
**WO 2014/128932 (28.08.2014 Gazette 2014/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Mitsubishi Electric Corporation Tokyo 100-8310 (JP)**

(72) Inventors:
• **ODAWARA, Shuhei**
  **Tokyo 100-8310 (JP)**

• **HARADA, Hisashi**
  **Tokyo 100-8310 (JP)**
• **IKEDA, Masahiro**
  **Tokyo 100-8310 (JP)**
• **SUGAHARA, Kengo**
  **Tokyo 100-8310 (JP)**

(74) Representative: **Sajda, Wolf E.**
  **Meissner, Bolte & Partner GbR**
  **Postfach 86 06 24**
  **81633 München (DE)**

(54) **PARTICLE THERAPY APPARATUS**

(57) A particle therapy apparatus has a rotating gantry (10) configured in such a way that an irradiation device (4) is rotated around a rotation axis ($X_R$) and a particle beam (B) is irradiated onto an irradiation subject; the rotating gantry (10) is provided with an entrance-side deflection electromagnet (1) having a deflection path ($1_C$) for radially deflecting the particle beam (B) supplied along the rotation axis ($X_R$) so as to guide the particle beam (B) to the irradiation device (4) and a straight path ($1_S$) that is switchable with the deflection path ($1_C$) and is for making the supplied particle beam (B) travel in a straight manner; there are provided trajectory correcting devices (5 and 6) having two position sensors that are arranged along the rotation axis ($X_R$) so as to flank the entrance-side deflection electromagnet (1).

Fig. 1

**Description**

Technical Field

The present invention relates to a particle therapy apparatus to be utilized in a cancer treatment or the like and particularly to a particle therapy apparatus in which multi-port irradiation is performed by use of a rotating gantry.

Background Art

In a particle beam therapy, the therapy is implemented by irradiating a charged particle beam (particle beam) onto a diseased site, which is a therapy subject, so as to cause a damage in the tissues of the diseased site. In this situation, in order to deliver a sufficient dose to the tissues of the diseased site without causing damage to the peripheral tissues thereof, it is required to appropriately control an irradiation dose and an irradiation coverage (irradiation field).

For that purpose, it is required to correctly maintain the trajectory of a particle beam; however, due to installation errors of apparatuses in the transport path between a radiation source and an irradiation device and an error in the magnetic-field intensity or the like, the particle-beam trajectory may be displaced from the designed trajectory.

On the other hand, in some cases, an irradiation method, which is referred to as a multi-port irradiation and in which particle beams are irradiated onto a diseased site while the irradiation directions thereof are changed from one another, is employed in a particle beam therapy, in order to secure the doses for the diseased site so as to maintain the therapy effect, while reducing doses to major organs.

As an apparatus to be utilized in multi-port irradiation, there is utilized a rotating gantry in which an irradiation device itself is rotated around a rotation axis including a diseased site (an isocenter). In the case of a particle therapy apparatus utilizing a rotating gantry, in order to prevent the state of an error with reference to the isocenter from fluctuating in accordance with the rotation angle (irradiation angle) of the rotating gantry, the accuracy of the trajectory is required, especially at the entrance of the rotating gantry.

Accordingly, it is conceivable that in the transport path, there is provided a trajectory correcting device, for example, as disclosed in Patent Document 1, in which the trajectory position and the gradient of a particle beam is corrected by use of two beam position sensors.

Prior Art Reference

Patent Documents

Patent Document 1 Japanese Patent Application Laid-Open JP 2003-282 300 A (paragraph 0036 and FIG. 1)
Patent Document 2 US Patent No. 4 917 344 (Column 3, Lines 20 through 61, FIG. 1a and 1b)

Disclosure of the Invention

Problems to be Solved by the Invention

However, in such a trajectory correcting device as described above, the accuracy is determined in accordance with the distance between the two beam position sensors; thus, it is required to form a predetermined-distance straight portion in the transport path. For example, in order to supply a particle beam, which has been on an accurate trajectory and passed through the entrance of a rotating gantry, to an irradiation device with an error of 0.1 mm or smaller, it is required to provide a straight portion of as long as several meters.

On top of that, when the dependence on the irradiation angle is considered, the straight portion needs to be provided on the rotation axis of the rotating gantry; therefore, not only is the installation space widened, but also the installation tolerance is restricted, and hence the plant may become excessively large.

The present invention has been implemented in order to solve the foregoing problems; the objective thereof is to obtain a particle therapy apparatus that suppresses the plant from becoming excessively large and can deliver an accurate and appropriate dose.

Means for Solving the Problems

A particle therapy apparatus according to the present invention has a rotating gantry configured in such a way that an irradiation device rotates around a rotation axis and a particle beam is irradiated onto an irradiation subject; the particle therapy apparatus is characterized in that the rotating gantry is provided with a deflection electromagnet having a deflection path for radially deflecting a particle beam supplied along the rotation axis so as to guide the particle beam to the irradiation device and a straight path that is switchable with the deflection path and is for making the supplied particle beam travel in a straight manner and in that there is provided a trajectory correcting device having two position sensors that are arranged along the rotation axis so as to flank the deflection electromagnet.

Advantages of the Invention

In a particle therapy apparatus according to the present invention, the straight distance is secured by use of the space inside a rotating gantry; therefore, there can be obtained a particle therapy apparatus that can suppress the installation space from becoming excessively large and can deliver an accurate and appropriate dose.

Brief Description of the Drawings

FIG. 1    is a view of the vicinity of a rotating gantry for explaining the configuration of a particle therapy apparatus according to Embodiment 1 of the present invention;

FIG. 2    is a view for explaining the overall configuration of the particle therapy apparatus according to Embodiment 1 of the present invention;

FIG. 3    is a view of the vicinity of a rotating gantry for explaining the configuration of a particle therapy apparatus according to Embodiment 2 of the present invention;

FIG. 4    is a view of the vicinity of a rotating gantry for explaining the configuration of a particle therapy apparatus according to Embodiment 3 of the present invention;

FIG. 5    is a view of the vicinity of a rotating gantry for explaining the configuration of a particle therapy apparatus according to Embodiment 4 of the present invention;

FIG. 6    is a view of the vicinity of a rotating gantry for explaining the configuration of a particle therapy apparatus according to Embodiment 5 of the present invention;

FIG. 7    is a set of schematic views that each illustrate the cross-sectional shape of a rotating gantry for explaining the configuration of a particle therapy apparatus according to Embodiment 6 of the present invention;

FIG. 8    is a set of views for comparing the size of a particle therapy apparatus according to Embodiment 7 of the present invention with the size of a conventional particle therapy apparatus; and

FIG. 9    is a set of views for comparing the size of a particle therapy apparatus according to Embodiment 8 of the present invention with the size of a conventional particle therapy apparatus.

Best Modes for Carrying Out the Invention

Embodiment 1

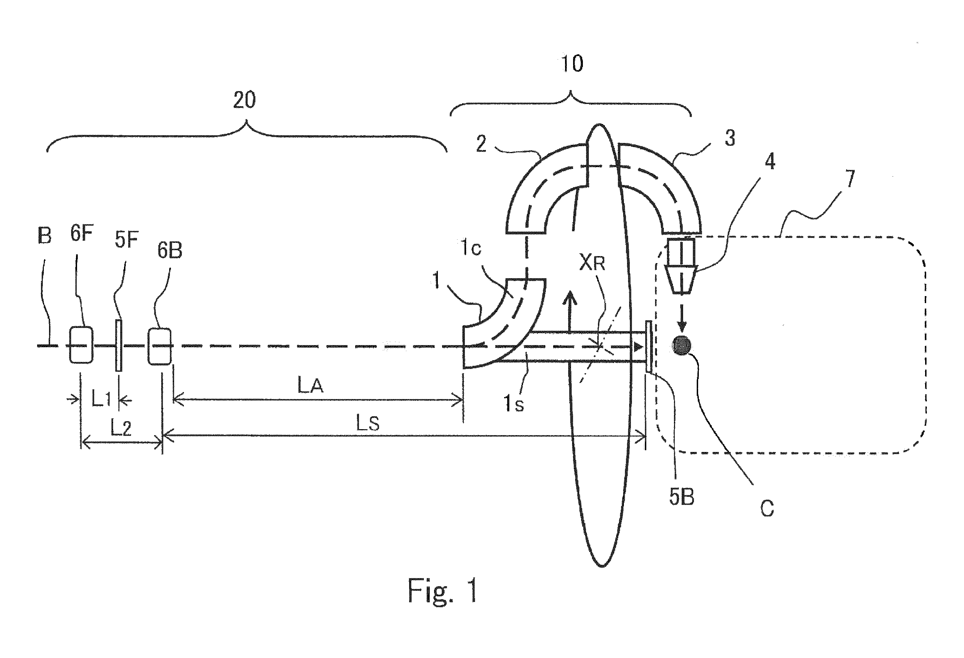

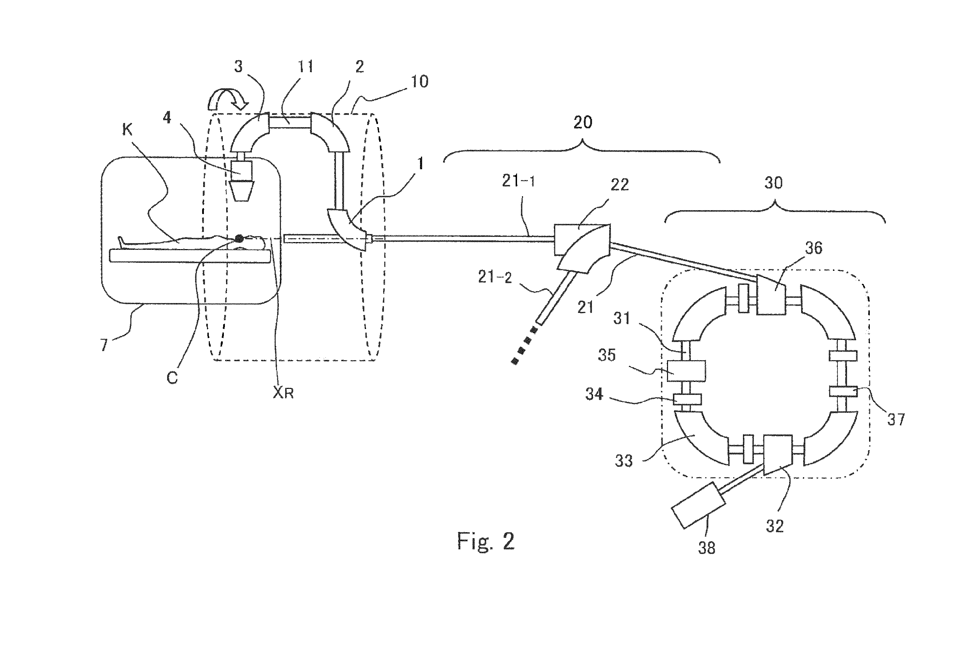

The configuration of a particle therapy apparatus according to Embodiment 1 of the present invention will be explained below. FIGS. 1 and 2 are views for explaining the configuration of a particle therapy apparatus according to Embodiment 1 of the present invention; FIG. 1 is a view illustrating the arrangement of major devices in the vicinity of a rotating gantry in the particle therapy apparatus.
FIG. 1 is a view for explaining the configuration of a device for correcting the trajectory of a particle beam to be supplied to the rotating gantry; FIG. 2 is a view for explaining the overall configuration of the particle therapy apparatus.
The characteristics of the particle therapy apparatus according to Embodiment 1 of the present invention are the configurations of deflection electromagnets, of the rotating gantry, that are to correct the trajectory of a particle beam to be supplied to the rotating gantry and the arrangement of beam position sensors for correcting the beam trajectory. However, before the explanation of the configurations and operations thereof, the overall configuration of the particle therapy apparatus will be explained with reference to FIG. 2.
In FIGS. 1 and 2, a particle therapy apparatus is provided with a circular accelerator (simply referred to as an accelerator 30, hereinafter), which is a synchrotron as the supply source (radiation source) of a particle beam; an irradiation device 4 provided in an irradiation chamber 7 having a rotating gantry; and a transport system 20 that connects the accelerator 30 and the irradiation device 4 and transports a particle beam from the accelerator 30 to the irradiation device 4.
The transport system 20 has transport paths $21_{-1}$, $21_{-2}$, $\cdots$, $21_{-n}$ (collectively referred to as a transport path 21) that are connected with two or more unillustrated irradiation chambers (irradiation devices thereof) including the irradiation chamber 7. In addition, by switching the trajectories through a switching electromagnet 22, a particle beam from the accelerator 30 can be supplied to an irradiation chamber where the particle beam is required. Other unillustrated irradiation chambers do not necessarily have the rotating gantry 10. Next, the configurations will be explained.

The accelerator 30 is provided with a vacuum duct 31, which is a trajectory path for making a charged particle circulate; an injector 32 for injecting a charged particle, supplied from a prestage accelerator 38, into the vacuum duct 31; a deflection electromagnet 33 for deflecting the trajectory of a charged particle so that the charged particle circulates along a circulation orbit inside the vacuum duct 31; a convergence electromagnet 34 that prevents a charged particle on the circular orbit from diverging and converges the charged particle; a high-frequency wave acceleration cavity 35 that applies a high-frequency voltage, synchronized with a circulating charged particle, to the circulating charged particle so as to accelerate the charged particle; a launching apparatus 36 that extracts a charged particle accelerated in the accelerator 30, as a particle beam having predetermined energy, from the accelerator 30 and launches the charged particle into the transport system 20; and a six-pole electromagnet 37 that excites resonance in the circular orbit so that the launching apparatus 36 launches a particle beam.

A charged particle in the circulation orbit is accelerated by a high-frequency-wave electric field; while being bent by a magnet, the charged particle is accelerated up to 70 % to 80 % of the light velocity and is launched into the transport system 20.

The transport system 20 is referred to as an HEBT (High Energy Beam Transport) system and is provided with a vacuum duct, which functions as a transport path for a particle beam, the switching electromagnet 22, which is a switching device that switches the trajectories of a particle beam, and a deflection electromagnet that deflects a particle beam at a predetermined angle.

The irradiation device 4 forms particle beams supplied from the transport system 20 into an irradiation field according to the size and the depth of an irradiation subject so as to irradiate the particle beams onto a diseased site. A particle beam to be supplied to the irradiation device 4 is a so-called pencil-shaped thin beam; the irradiation device 4 is provided with a scanning electromagnet that deflects the beam in an arbitrary direction on a plane that is approximately perpendicular to the beam axis, a ridge filter for enlarging the width of a Bragg peak in accordance with the thickness of an irradiation subject, a range shifter for changing the energy (range) of a particle beam in accordance with the depth (irradiation depth) of the irradiation subject, and the like.

The irradiation device 4 is provided in the rotating gantry 10 that rotates on a rotation axis $X_R$ including the center (isocenter C) of an irradiation subject. In the rotating gantry 10, there are provided an entrance-side deflection electromagnet 1 that is disposed on the rotation axis $X_R$ and deflects a particle beam, supplied from the transport system 20 onto the rotation axis $X_R$, radially outward; an intermediate deflection electromagnet 2 that deflects a particle beam, which has been made to travel radially outward by the entrance-side deflection electromagnet 1, in a direction having the component of the rotation axis $X_R$; and an exit-side deflection electromagnet 3 that deflects a particle beam, which has been made to travel toward the rotation axis $X_R$ by the intermediate deflection electromagnet 2, in such a way that the particle beam travels radially inward, i.e., toward the rotation axis $X_R$; and a beam transport pipe 11 that connects the deflection electromagnets (1 through 3) with the irradiation device 4.

Accordingly, the irradiation device 4 always points the irradiation direction to the rotation axis $X_R$, even when the irradiation direction changes due to the rotation of the rotating gantry 10. In contrast, a treatment table, on which a patient K inside an irradiation chamber 7 is placed, and the like are fixed regardless of rotation of the rotating gantry 10; therefore, the irradiation device 4 can irradiate a particle beam formed in accordance with a diseased site onto the diseased site at an arbitrary angle.

However, because as described in "Background Art", the irradiation angle can be changed by use of the rotating gantry 10, the effect, at the irradiation device 4, of the trajectory displacement of a particle beam to be supplied to the entrance of the rotating gantry 10, i.e., the entrance-side deflection electromagnet 1 differs depending on the rotation angle.

Thus, it is conceivable that as disclosed in Patent Document 1, a trajectory correcting device, which is configured with two pieces of beam position sensors and two pairs of steering electromagnets and corrects the position and the gradient of the trajectory of a particle beam, is provided in the transport path.

In this trajectory correcting device, a pair of steering electromagnets are arranged at the uppermost stream position of the beam trajectory, one piece of beam position sensor is disposed at the lowermost stream position, and the remaining steering electromagnets and beam position sensor are arranged with reduced space in such a way as to be on a straight line between the uppermost-stream steering electromagnets and the lowermost-stream position sensor.

In the case where the trajectory of a particle beam to be supplied to the rotating gantry is corrected, the two pieces of beam position sensors and two pairs of steering electromagnets are placed in an extended line of the rotation axis $X_R$ of the rotating gantry 10, as illustrated in FIG. 1.

In FIG. 1, the uppermost-stream steering electromagnet and the lowermost-stream position sensor will be referred to as a first steering electromagnet 6F and a second position sensor 5B, respectively. In addition, the steering electromagnet and the beam position sensor that are arranged between the first steering electromagnet and the second position sensor will be referred to as a second steering electromagnet 6B and a first position sensor 5F, respectively.

The reason why in the above explanation, the expression "... pairs of steering electromagnets" is utilized is that in each of the steering electromagnets, there are combined two deflection electromagnets that each deflect a particle beam B in the respective directions corresponding to two directions (for example, the x direction and the y direction that are perpendicular to each other) that cross each other on a plane that is perpendicular to the traveling direction of the particle beam B.

In contrast, as far as the beam position sensor is concerned, the expression "... pieces of" is utilized because in general, a beam position sensor can measure two directions.

There will be explained a trajectory correction that is performed by such a trajectory correcting device in which the first steering electromagnet 6F and the second steering electromagnet 6B (collectively referred to as a steering electromagnet 6) and the first position sensor 5F and the second position sensor 5B (collectively referred to as a beam position sensor 5) are arranged on a straight line.

The first steering electromagnet 6F changes the traveling direction of a beam in such a way that the trajectory position of the particle beam B detected by the first position sensor 5F coincides with the designed position. Then, the second steering electromagnet 6B changes the traveling direction of a beam in such a way that the beam position detected by the second position sensor 5B coincides with the beam position that has been detected by the first position sensor 5F.

In other words, the first steering electromagnet 6F deflects the particle beam B so as to correct the trajectory position of the particle beam B; the second steering electromagnet 6B deflects the particle beam B so as to correct the gradient of the trajectory of the particle beam B. As a result, the trajectory position and the gradient of the particle beam B that passed through the second position sensor 5B can be corrected.

In this situation, the accuracy of the trajectory of the particle beam B that passes through the second position sensor 5B largely depends on the distance $L_S$ between the second steering electromagnet 6B (strictly speaking, the position at which deflection is implemented) and the second position sensor 5B. Letting $\Delta x_{5B}$ and $S_B$ denote the displacement amount of the trajectory, at the second position sensor 5B, from the trajectory at the second steering electromagnet 6B and the trajectory gradient between the second steering electromagnet 6B and the second position sensor 5B, respectively, the displacement amount of the trajectory at the second position sensor 5B can be expressed as the Equation (1).

$$\Delta x_{5B} = S_B \times L_S \qquad \cdot\cdot(1).$$

When it is assumed that the position measurement resolution (error) of the beam position sensor 5 is $\Delta_E$, it is should considered that the amount of trajectory displacement $\Delta x_{5B}$ at the second position sensor 5B is maximally $\Delta_E$, even when the trajectory can accurately be corrected within the range of the resolution of the beam position sensor 5. That is to say, it should be considered that the gradient $S_B$ includes the error $\Delta S_B$ given by the Equation (2).

$$\Delta S_B = \Delta_E / L_S \qquad \cdot\cdot\cdot(2).$$

In this situation, in the case where the trajectory errors at two points on the beam transport system 20 are considered, the trajectory errors $\Delta x_1$ and $\Delta x'_1$ at downstream points can be expressed by a linear combination of the trajectory errors $\Delta x_0$ and $\Delta x'_0$ at upstream points, as represented in the Equation (3).

$$\begin{pmatrix} \Delta x_1 \\ \Delta x'_1 \end{pmatrix} = M \begin{pmatrix} \Delta x_0 \\ \Delta x'_0 \end{pmatrix} \qquad \cdot\cdot\cdot(3)$$

where $\Delta x_0$ and $\Delta x_1$ are errors in the positions of trajectories, and $\Delta x'_0$ and $\Delta x'_1$ are errors in the gradients of trajectories; M is referred to as a transport matrix, which is a square matrix representing the transport between two points.

Because it is the same as the error in the trajectory gradient at the second position sensor 5B, the error in the trajectory gradient at the entrance of the rotating gantry 10 is $\Delta S_B$. In this situation, the error $\Delta x_4$ caused by $\Delta S_B$, among the errors in the trajectory positions in the section between the entrance of the rotating gantry 10 and the irradiation device 4 can be expressed as the Equation (4) by use of the first-row second-column component $M_{12}$ of the transport matrix for the section between the entrance of the rotating gantry 10 and the irradiation device 4.

$$\Delta x_4 = M_{12} \Delta S_B \qquad \cdot\cdot\cdot(4).$$

$M_{12}$ is a value that changes depending on the configuration of the rotating gantry 10; for example, with a given configuration, $M_{12}$ becomes 1.4. When the rotating gantry 10 is configured in such a way that $M_{12}$ becomes 1.4 and it is desired to suppress the error $\Delta x_4$ at the irradiation device 4 from exceeding 0.1 or so, for example, it is required to set $\Delta S_B$ to at least 0.07 ppm or so. In this situation, for example, when the position detection accuracy $\Delta_E$ of the beam position sensor 5 is 10 $\mu$m, the Equation (2) suggests that $L_S$ may be 14 cm or so.

However, because an actual particle-beam intensity has a distribution (e.g., Gaussian distribution) with respect to the beam axis, it is difficult to accurately detect the center of a beam, and hence the position detection accuracy is several hundreds micrometers or so.

With the position detection accuracy of this level, in order to suppress the position displacement of a particle beam traveling through a path of several meters from exceeding 0.5 mm, for example, it is required to set the distance $L_S$ between the second steering electromagnet 6B and the second position sensor 5B to several meters or longer.

Furthermore, as described above, it is required to secure the distance $L_S$ on the rotation axis $X_R$ of the rotating gantry 10. Accordingly, not only becomes the installation space larger, but also the installation tolerance is restricted, and hence the plant may become excessively large.

Thus, in the particle therapy apparatus according to Embodiment 1 of the present invention, the straight portion for increasing the distance $L_S$ expands across the entrance of the rotating gantry 10. Specifically, in addition to a normal deflection path $1_C$ that deflects the particle beam B radially outward, a straight path $1_S$ that does not deflect the incident particle beam B but makes it travel straightly is provided in the entrance-side deflection electromagnet 1, which is the entrance of the rotating gantry 10.

An unillustrated vacuum duct provided in the entrance-side deflection electromagnet 1 is configured in such a way as to ramify into the deflection direction (deflection path $1_C$) and the straight line direction (straight path $1_S$) whose center is the rotation axis $X_R$; switching to an arbitrary path is performed by an unillustrated control unit.

In addition, the second position sensor 5B is disposed on the extended line from the straight-line-direction straight path $1_S$; a vacuum duct secures a vacuum atmosphere also in the path leading to the second position sensor 5B. In the second position sensor 5B provided on the outside wall face of the irradiation chamber 7, there is provided a shielding wall in order to prevent the particle beam B from leaking from the downstream side thereof.

When a particle beam is irradiated, the normal deflection path $1_C$ is utilized; when in order to correct the trajectory, the steering electromagnet 6 is adjusted, the path of the entrance-side deflection electromagnet 1 is switched to the straight path $1_S$. When the path of the entrance-side deflection electromagnet 1 is switched to the straight path $1_S$, the entrance-side deflection electromagnet 1 is not energized so that the particle beam B reaches the second position sensor 5B without being deflected.

In this regard, however, it may be allowed that in order to cancel the residual magnetic field of the entrance-side deflection electromagnet 1, the auxiliary coil, wound in the same manner as the main coil (unillustrated) of the entrance-side deflection electromagnet 1, is energized.

As a result, in the case where the entrance-side deflection electromagnet 1 (strictly speaking, the main coil) is not energized, it can be regarded that between the second position sensor 5B and the second steering electromagnet 6B, there exists no element that changes the beam traveling direction.

Accordingly, when it is considered that the second steering electromagnet 6B and the second position sensor 5B are arranged on the same straight line along the rotation axis $X_R$, it can be regarded that the gradient of the trajectory, between the second steering electromagnet 6B and the second position sensor 5B, that expands, on the way, across the entrance of the rotating gantry 10 is the same as the gradient of the trajectory in the path that passes through the normal deflection path $1_C$ and then reaches the irradiation device 4.

Thus, part of the distance $L_S$ between the two beam position sensors 5 can be increased by the distance between the entrance of the rotating gantry 10 and the wall face, of the irradiation chamber 7, on which the second position sensor 5B is provided.

Accordingly, it is made possible that with the accuracy of the trajectory gradient of the particle beam B maintained, the distance $L_A$ to the entrance of the rotating gantry 10 in the straight portion in which the trajectory correcting devices (5 and 6) are arranged can be shortened.

The above calculation of distances and positional displacements has been explained under the assumption that the position of the trajectory detected by the first position sensor 5F and the position of the trajectory that passes through the second steering electromagnet 6B are the same; however, strictly speaking, such an error as described below is included.

For example, letting $L_1$, $L_2$, and $\Delta x_{6u}$ denote the distance between the first steering electromagnet 6F and the first position sensor 5F, the distance between the first steering electromagnet 6F and the second steering electromagnet 6B, and the amount of trajectory displacement at the first steering electromagnet 6F, in the case where at the first position sensor 5F, the particle beam B passes through a trajectory that is the same (the displacement amount: 0) as designed one, the trajectory of the particle beam B that passes through the second steering electromagnet 6B includes, as represented by the Equation (5), the displacement amount $\Delta x_{6d}$ corresponding to the interior division of the distance

between the devices.

$$\Delta x_{6d} = \Delta x_{6u} \times (L_2 - L_1)/L_2 \qquad \cdots (5).$$

In this situation, when the distance between the first steering electromagnet 6F and the second steering electromagnet 6B or the distance between the first steering electromagnet 6F and the first position sensor 5F is set to be sufficiently long in comparison with the distance ($L_2 - L_1$) between the second steering electromagnet 6B and the first position sensor 5F, the displacement amount $\Delta x_{6d}$ can be neglected.

Alternatively, for the purpose that the position of the particle beam B at the time it passes through the second steering electromagnet 6B coincides with the designed position, the position thereof at the first position sensor 5F may be corrected so as to shift from the designed value, by use of the relationship represented by the Equation (5) and values converted through the excitation value at the first steering electromagnet 6F.

It goes without saying that an arbitrary device including various kinds of electromagnets may be inserted into the section between the first steering electromagnet 6F and the second steering electromagnet 6B. It is more desirable that in the above method, arrangement considering the foregoing errors is implemented.

As described above, the particle therapy apparatus according to Embodiment 1 has the irradiation device 4 that forms the particle beam B into an irradiation field corresponding to an irradiation subject and then irradiates the particle beam B and the rotating gantry 10 configured in such a way that the particle beam B is irradiated onto the irradiation subject while the irradiation device 4 is rotated around the rotation axis $X_R$.

The particle therapy apparatus is configured in such a way that in the rotating gantry 10, there is provided a deflection electromagnet (the entrance-side deflection electromagnet 1) having the deflection path $1_C$ for radially deflecting the particle beam B, supplied along the rotation axis $X_R$, so as to guide the particle beam B to the irradiation device 4 and the straight path $1_S$ that is switchable with the deflection path $1_C$ and is to make the supplied particle beam B travel in a straight manner, in such a way that there are provided the first position sensor 5F that detects the trajectory position of the particle beam B to be supplied to the rotating gantry 10, the first steering electromagnet 6F that is provided at the upstream side of the first position sensor 5F and corrects the trajectory position of the particle beam B that passes through the first position sensor 5F, the second steering electromagnet 6B that is provided at the downstream side of the first position sensor 5F and corrects the trajectory gradient $S_B$ of the particle beam B whose trajectory position has been corrected by the first steering electromagnet 6F, and the second position sensor 5B that is provided at a position that is at the downstream side of the second steering electromagnet 6B and a predetermine distance $L_S$ away from the second steering electromagnet 6B and that detects the trajectory position of the particle beam B so as to correct the trajectory gradient $S_B$ caused by the second steering electromagnet 6B, and in such a way as to include the trajectory correcting devices (5 and 6) in which the first position sensor 5F and the second position sensor 5B are arranged along the rotation axis $X_R$ so as to flank a deflection electromagnet (the entrance-side deflection electromagnet 1).

As a result, even when the entrance-side straight distance of the rotating gantry 10 is shortened, the distance $L_S$ between the second steering electromagnet 6B and the second position sensor 5B can be secured by a predetermined distance; therefore, the plant is suppressed from becoming excessively large, and the accuracy of the gradient $S_B$ of the particle beam B is raised; thus, a particle therapy apparatus that can deliver an accurate and appropriate dose can be obtained.

Embodiment 2

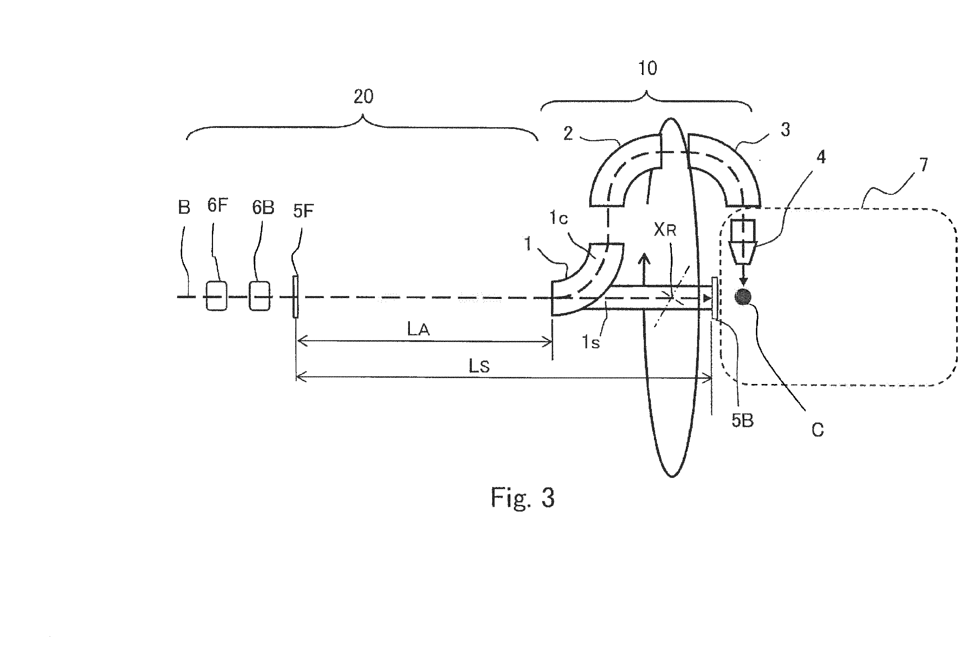

In Embodiment 2, the arrangement order, in Embodiment 1, of the beam position sensors and the steering electromagnets included in the trajectory correcting devices is partially changed. FIG. 3 is to explain a particle therapy apparatus according to Embodiment 2; FIG. 3 is a view illustrating the arrangement of major devices in the vicinity of a rotating gantry in the particle therapy apparatus, and is to explain the configuration of a device for correcting the trajectory of a particle beam to be supplied to the rotating gantry.

In FIG. 3, constituent elements that are the same as those in Embodiment 1 are designated by the same reference characters, and the detailed explanations therefor will be omitted. FIG. 2 explained in Embodiment 1 is also utilized in Embodiments after and including Embodiment 2.

As illustrated in FIG. 3, also in Embodiment 2, the first steering electromagnet 6F and the second position sensor 5B are arranged at the uppermost stream side and at the most downstream side, respectively, along the traveling direction of the particle beam B, and the second steering electromagnet 6B and the first position sensor 5F are arranged between the first steering electromagnet 6F and the second position sensor 5B.

The foregoing devices are arranged on the rotation axis $X_R$ in such a way that the entrance of the rotating gantry 10 is situated between the second position sensor 5B and the second steering electromagnet 6B/the first positon sensor 5F. In this regard, however, Embodiment 2 differs from Embodiment 1 in that the second steering electromagnet 6B is disposed at the upstream side of the first position sensor 5F.

In Embodiment 2, in order to reduce the errors in the trajectory positon and the gradient of the particle beam B that passes through the entrance of the rotating gantry 10, the deflection amounts of the two steering electromagnets 6 (the first steering electromagnet 6F and the second steering electromagnet 6B) are adjusted so that the trajectory positions of the particle beam B becomes the respective designed position at both the beam position sensors 5 (the first position sensor 5F and the second position sensor 5B).

In this situation, as is the case with Embodiment 1, the entrance-side deflection electromagnet 1 is not energized so that in the section between the second steering electromagnet 6B and the second position sensor 5B, there exists no element that changes the traveling direction of the particle beam B.

Also in this case, when instead of the distance between the second steering electromagnet 6B and the second position sensor 5B, the distance $L_S$ between the first position sensor 5F and the second position sensor 5B is utilized, the longer is the distance $L_S$, the smaller becomes the positional displacement $\Delta x_4$ at the irradiation device 4.

Accordingly, when the straight portion between the second steering electromagnet 6B and the second position sensor 5B expands across the entrance of the rotating gantry 10, the distance $L_A$ to the entrance of the rotating gantry 10 in the straight portion in which the trajectory correcting devices (5 and 6) are arranged can be shortened with the accuracy of the trajectory gradient of the particle beam B maintained.

Moreover, in this arrangement, the particle beam B whose position and gradient have been corrected by the two steering electromagnets 6 passes through the two beam position sensors 5; therefore, it is not required to consider such an error as represented in the Equation (5) that has been explained in Embodiment 1. In other words, it is only necessary that the trajectory position of the particle beam B that passes through the two beam position sensors 5 is made to coincide only with the designed value.

It may be allowed that an arbitrary device including various kinds of electromagnets is inserted into the section between the first steering electromagnet 6F and the first position sensor 5F; even in that situation, unlike Embodiment 1, it is not required to consider the device arrangement that reduces the error represented by the Equation (5).

As described above, the particle therapy apparatus according to Embodiment 2 has the irradiation device 4 that forms the particle beam B into an irradiation field corresponding to an irradiation subject and then irradiates the particle beam B and the rotating gantry 10 configured in such a way that the particle beam B is irradiated onto the irradiation subject while the irradiation device 4 is rotated around the rotation axis $X_R$.

The particle therapy apparatus is configured in such a way that in the rotating gantry 10, there is provided a deflection electromagnet (the entrance-side deflection electromagnet 1) having the deflection path $1_C$ for radially deflecting the particle beam B, supplied along the rotation axis $X_R$, so as to guide the particle beam B to the irradiation device 4 and the straight path $1_S$ that is switchable with the deflection path $1_C$ and is to make the supplied particle beam B travel in a straight manner, in such a way that there are provided the first position sensor 5F that detects the trajectory position of the particle beam B to be supplied to the rotating gantry 10, the first steering electromagnet 6F that is provided at the upstream side of the first position sensor 5F and corrects the trajectory position of the particle beam B that passes through the first position sensor 5F, the second steering electromagnet 6B that is provided in the section between the first steering electromagnet 6F and the first position sensor 5F and corrects the trajectory gradient $S_B$ of the particle beam B whose trajectory position has been corrected by the first steering electromagnet 6F, and the second position sensor 5B that is provided at a position that is at the downstream side of the first position sensor 5F and a predetermine distance $L_S$ away from the first position sensor 5F and that detects the trajectory position of the particle beam B so as to correct the trajectory gradient $S_B$ caused by the second steering electromagnet 6B, and in such a way as to include the trajectory correcting devices (5 and 6) in which the first position sensor 5F and the second position sensor 5B are arranged along the rotation axis $X_R$ so as to flank a deflection electromagnet (the entrance-side deflection electromagnet 1).

As a result, even when the entrance-side straight distance of the rotating gantry 10 is shortened, the distance $L_S$ between the first position sensor 5F and the second position sensor 5B can be secured by a predetermined distance; therefore, the plant is suppressed from becoming excessively large, and the accuracy of the trajectory gradient $S_B$ of the particle beam B is raised; thus, a particle therapy apparatus that can deliver an accurate and appropriate dose can be obtained.

As described in Embodiments 1 and 2, either the order of the arrangement of the first position sensor 5F and the second steering electromagnet 6B or the distance relation between them may be considered; i.e., it is only necessary to pay attention to the two beam position sensors 5. In other words, a particle therapy apparatus according to any one of Embodiments 1 and 2 has the rotating gantry 10 configured in such a way that the particle beam B is irradiated onto the irradiation subject while the irradiation device 4 is rotated around the rotation axis $X_R$.

The particle therapy apparatus is configured in such a way that in the rotating gantry 10, there is provided a deflection electromagnet (the entrance-side deflection electromagnet 1) having the deflection path $1_C$ for radially deflecting the particle beam B, supplied along the rotation axis $X_R$, so as to guide the particle beam B to the irradiation device 4 and the straight path $1_S$ that is switchable with the deflection path $1_C$ and is to make the supplied particle beam B travel in a straight manner, and in such a way as to include the trajectory correcting devices (5 and 6) in which the two position sensor (beam position sensors 5) are arranged along the rotation axis $X_R$ so as to flank a deflection electromagnet (the entrance-side deflection electromagnet 1).

As a result, even when the straight distance $L_A$ at the entrance side of the rotating gantry 10 is shortened, the distance $L_S$ between the second steering electromagnet 6B and the second position sensor 5B or between the first position sensor 5F and the second position sensor 5B can be secured by a predetermined distance; therefore, the plant is suppressed from becoming excessively large, and the accuracy of the trajectory gradient $S_B$ of the particle beam B is raised; thus, a particle therapy apparatus that can deliver an accurate and appropriate dose can be obtained.

Embodiment 3

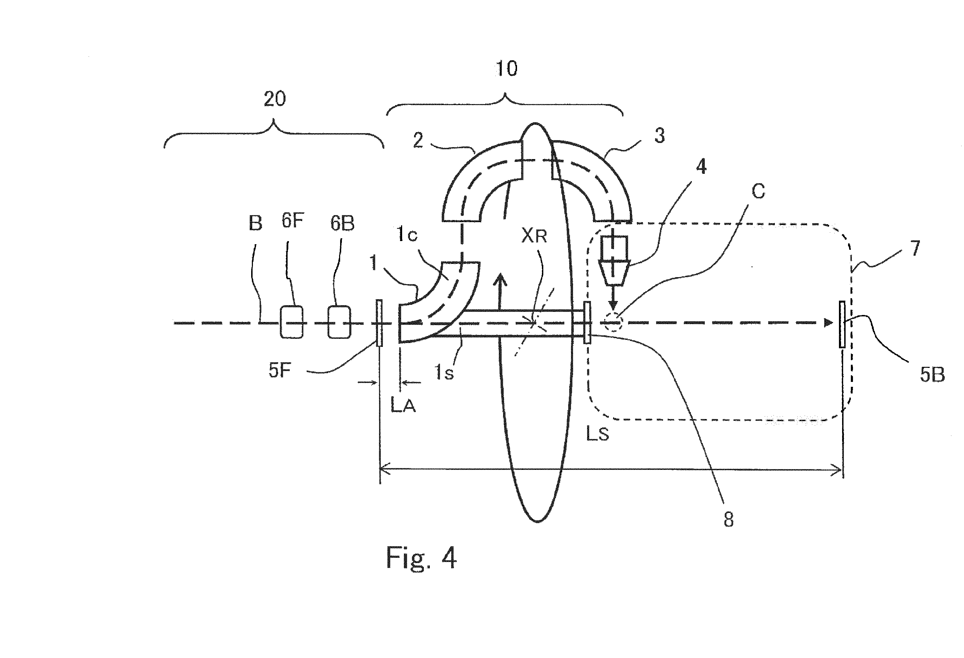

In each of Embodiments 1 and 2, an example had been described in which the straight portion up to the second position sensor is extended to the front of an irradiation chamber. In Embodiment 3 and Embodiments 4 and 5 to be described later, the straight portion up to the second position sensor is extended to the inside of the irradiation chamber. FIG. 4 is to explain a particle therapy apparatus according to Embodiment 3; FIG. 4 is a view illustrating the arrangement of major devices in the vicinity of a rotating gantry in the particle therapy apparatus, and is to explain the configuration of a device for correcting the trajectory of the particle beam B to be supplied to the rotating gantry.

In FIG. 4, constituent elements that are the same as those in Embodiment 1 or Embodiment 2 are designated by the same reference characters, and the detailed explanations therefor will be omitted. FIG. 2 explained in Embodiment 1 is also utilized in Embodiment 3 and Embodiments 4 and 5, described later.

As illustrated in FIG. 4, in a particle therapy apparatus according to Embodiment 3, a vacuum window 8 for making the particle beam B penetrate therethrough is provided at the front portion of the straight path $1_S$ of the entrance-side deflection electromagnet 1, i.e., at the portion corresponding to the wall of the irradiation chamber 7.

In addition, the second position sensor 5B is provided on the extended line of the straight path $1_S$ inside the irradiation chamber 7. As a result, the particle beam B that has traveled on the straight path $1_S$ advances to the second position sensor 5B provided inside the irradiation chamber 7, through the vacuum window 8.

Accordingly, in the case where the trajectory of the particle beam B is corrected, any person including the patient K is prevented from entering the irradiation chamber 7, and any obstacle is prevented from existing in the straight line between the vacuum window 8 and the second position sensor 5B.

As a result, even though there exists atmospheric air in the space between the vacuum window 8 and the second position sensor 5B, the space inside the irradiation chamber 7 is utilized, so that the distance $L_S$ between the first position sensor 5F and the second position sensor 5B can be increased.

The arrangement of increasing the distance $L_S$ by utilizing the space inside the irradiation chamber 7 can be applied also to the case where the first position sensor 5F is disposed at the upstream side of the second steering electromagnet 6B. That is to say, because the straight portion between the first position sensor 5F and the second position sensor 5B or between the second steering electromagnet 6B and the second position sensor 5B is provided in the space inside the irradiation chamber 7, the distance $L_A$ between the first position sensor 5F and the entrance of the rotating gantry 10 or between the second steering electromagnet 6B and the entrance of the rotating gantry 10 can further be shortened in comparison with the distance $L_A$ in each of Embodiments 1 and 2.

It goes without saying that when a treatment is performed or a person enters the irradiation chamber 7, the particle beam B is blocked from leaking out of the vacuum window 8 even when the setting has been made in such a way that the particle beam B passes through the deflection path $1_C$.

As described above, in the particle therapy apparatus according to Embodiment 3, the second position sensor 5B, among the two beam position sensors 5, that is disposed at the downstream side is provided inside the irradiation chamber 7 for irradiating the particle beam B; therefore, the distance $L_A$ between the first position sensor 5F and the entrance of the rotating gantry 10 or between the second steering electromagnet 6B and the entrance of the rotating gantry 10 can further be shortened.

Embodiment 4

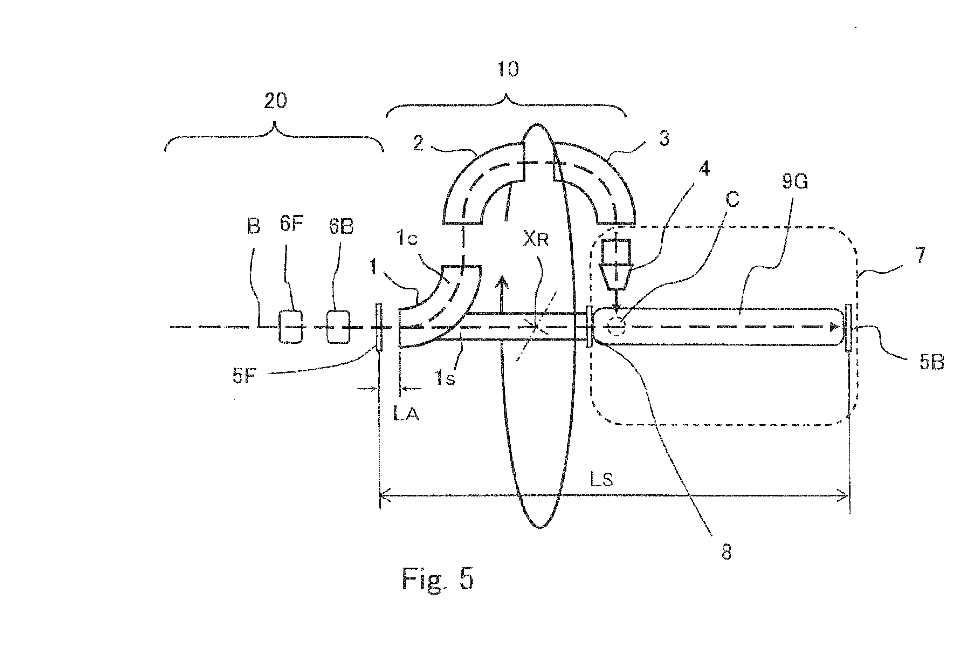

In Embodiment 4, in comparison with foregoing Embodiment 3, there is provided a bag-shaped duct, which is filled with, for example, a predetermined gas and can readily be detached, is provided in the space between the vacuum window and the second position sensor. FIG. 5 is to explain a particle therapy apparatus according to Embodiment 4.

FIG. 5 is a view illustrating the arrangement of major devices in the vicinity of a rotating gantry in the particle therapy apparatus, and is to explain the configuration of a device for correcting the trajectory of a particle beam to be supplied to the rotating gantry. In FIG. 5, constituent elements that are the same as those in Embodiment 3 are designated by the same reference characters, and the detailed explanations therefor will be omitted.

As illustrated in FIG. 5, in the particle therapy apparatus according to Embodiment 4, there is provided a bag (bag container for containing gas)-shaped duct 9G, which is filled with a small-atomic-number gas such as helium and can readily be detached, is provided on the trajectory of the particle beam B in the space between the vacuum window 8 and the second position sensor 5B.

As a result, because the spread of a beam due to atmospheric scattering can be reduced, a thin beam including no effect of the atmospheric scattering can reach the second position sensor 5B. Accordingly, in comparison with Embodiment 3, the accuracy of detecting the beam position at the second position sensor 5B is raised and hence the trajectory of the particle beam B can more accurately be corrected. When a treatment is performed (in the case where the particle beam B passes through the deflection path 1C), the duct 9G is removed.

As described above, in the particle therapy apparatus according to Embodiment 4, the attachable and detachable duct 9G whose inside can be filled with an atmosphere of a predetermine gas is provided in the inside of the irradiation chamber 7 in the space between the entrance-side deflection electromagnet 1 and the second position sensor 5B; therefore, the accuracy of detecting the beam position at the second position sensor 5B is raised and hence the trajectory of the particle beam B can more accurately be corrected.

Embodiment 5

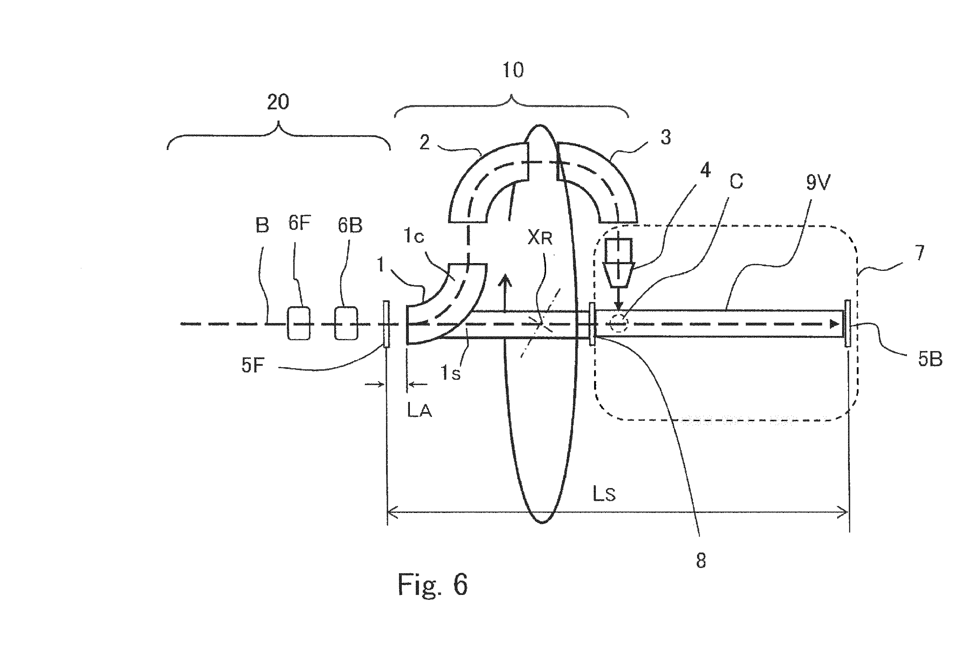

In Embodiment 5, in contrast to Embodiment 4, a vacuum duct is provided instead of the bag-shaped duct for maintaining a gas atmosphere. FIG. 6 is to explain a particle therapy apparatus according to Embodiment 5.

FIG. 6 is a view illustrating the arrangement of major devices in the vicinity of a rotating gantry in the particle therapy apparatus, and is to explain the configuration of a device for correcting the trajectory of the particle beam B to be supplied to the rotating gantry. In FIG. 6, constituent elements that are the same as those in Embodiment 4 are designated by the same reference characters, and the detailed explanations therefor will be omitted.

As illustrated in FIG. 6, in the particle therapy apparatus according to Embodiment 5, a vacuum duct 9V is provided on the trajectory, of the particle beam B, between the vacuum window 8 and the second position sensor 5B. As a result, because the spread of a beam due to atmospheric scattering can be reduced, a thin beam including no effect of the atmospheric scattering can reach the second position sensor 5B.

Accordingly, as is the case with Embodiment 4, the accuracy of detecting the beam position at the second position sensor 5B is raised and hence the trajectory of the particle beam B can more accurately be corrected. As is the case with Embodiment 4, when a treatment is performed (when the particle beam B passes through the deflection path $1_C$), the vacuum duct 9V is removed also in Embodiment 5.

As described above, in the particle therapy apparatus according to Embodiment 5, the attachable and detachable duct 9V whose inside can be evacuated is provided in the inside of the irradiation chamber 7 in the space between the entrance-side deflection electromagnet 1 and the second position sensor 5B; therefore, the accuracy of detecting the trajectory position of the particle beam B at the second position sensor 5B is raised and hence the trajectory of the particle beam B can more accurately be corrected.

Embodiment 6

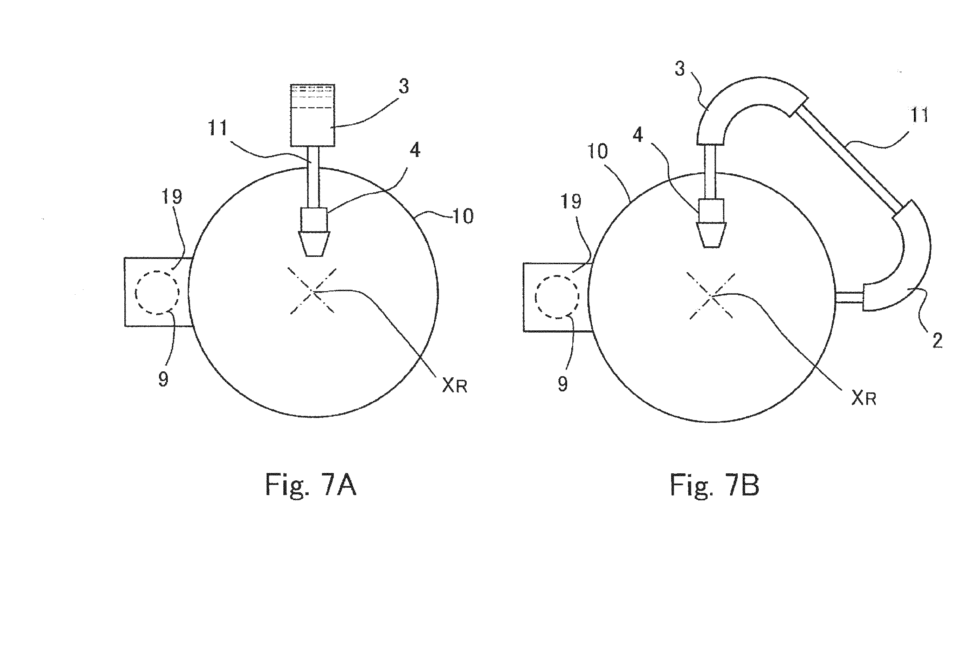

In Embodiment 6, in contrast to each of Embodiments 4 and 5, a containing unit for storing a duct is provided on a rotating gantry. FIGS. 7(a) and 7(b) are views for explaining respective particle therapy apparatuses according to Embodiment 6 and are schematic views illustrating the cross-sectional shapes in a direction perpendicular to the rotation axis in respective rotating gantries of different types.

In FIGS. 7(a) and 7(b), constituent elements that are the same as those in Embodiment 4 or Embodiment 5 are designated by the same reference characters, and the detailed explanations therefor will be omitted.

As illustrated in FIG. 7(a) or 7(b), in the particle therapy apparatus according to Embodiment 6, the end portion, of the duct 9G or the vacuum duct 9V (collectively, referred to as a duct 9), at the beam-traveling-direction side is contained in the rotating gantry 10. In addition, a containing unit 19 is provided in the region excluding the irradiation device 4 and the position that is diagonal to the irradiation device 4 in the outer circumference direction of the rotating gantry 10 and excluding the region where the intermediate deflection electromagnet 2 and the exit-side deflection electromagnet 3 of the rotating gantry 10 and the beam transport pipe 11 exist.

In the case where Embodiment 6 is applied to the rotating gantry 10 (refer to FIG. 7(b)) as disclosed in Patent Document 2, attention is paid because the irradiation device 4, the intermediate deflection electromagnet 2, the exit-side deflection electromagnet 3, and the beam transport pipe 11 do not exist in the same azimuth region.

When a treatment is performed, the duct 9G or the vacuum duct 9V is contained in the containing unit 19. As a result, it is not required to introduce the duct 9G or the vacuum duct 9V into or drain it from the irradiation chamber 7; thus, the time required for preparing the treatment can be shortened.

As described above, in the particle therapy apparatus according to Embodiment 6, because the containing unit 19 for containing the duct 9 is provided on the rotating gantry 10, the time required for preparing a treatment can be shortened.

Embodiment 7

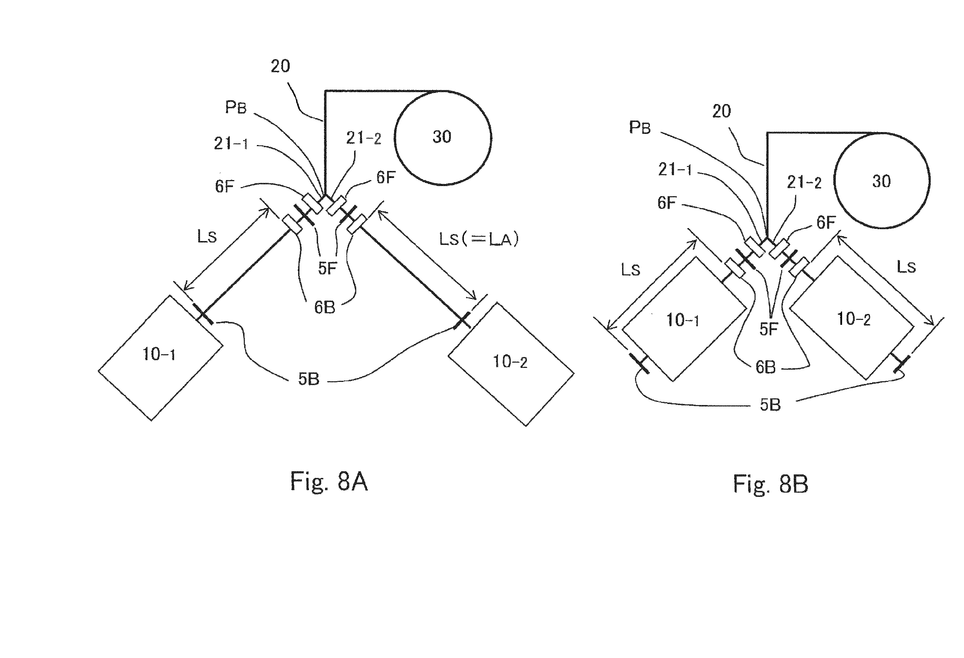

A particle therapy apparatus according to Embodiment 7 is the one to which technologies according to foregoing embodiments are applied and in which two rotating gantries are arranged symmetrically with each other. FIGS. 8(a) and 8(b) are views for comparing the plant size of a conventional particle therapy apparatus with the plant size of a particle therapy apparatus according to Embodiment 7.

FIG. 8(a) and FIG. 8(b) illustrate the plant of a conventional example and the plant of the particle therapy apparatus according to Embodiment 7, respectively. In FIGS. 8(a) and 8(b), constituent elements that are the same as those in Embodiments 1 through 6 are designated by the same reference characters, and the detailed explanations therefor will be omitted.

As illustrated in FIGS. 8(a) and 8(b), each of the conventional example and the particle therapy apparatus according to Embodiment 7, the transport system 20 communicating with the accelerator 30 is divided, at a branch point $P_B$, into transport paths $21_{-1}$ and $21_{-2}$ communicating with rotating gantries $10_{-1}$ and $10_{-2}$, respectively; the gantries $10_{-1}$ and $10_{-2}$ are arranged symmetrically with each other.

In the case where as in the past, it is required to secure the distance $L_S$ of the straight portion between the second steering electromagnet 6B and second position sensor 5B before the entrance of the rotating gantry 10, it is necessary to dispose the rotating gantry 10 in such a way that the distance between the branch point $P_B$ and the entrance of the rotating gantry 10 is longer than the distance $L_S$.

In contrast, in the case where as described in Embodiment 7, the straight portion between the two beam position sensor 5 is formed in such a way as to flank the entrance of the rotating gantry 10, the rotating gantry 10 can be disposed in such a way that the distance between the branch point $P_B$ and the entrance thereof is shorter than the distance $L_S$; thus, the area required for installing the plant can be reduced.

This example has explained the arrangement of the respective groups of rotating gantries and peripheral devices that are arranged symmetrically with each other; however, it goes without saying that an irradiation chamber of another type may exist.

Embodiment 8

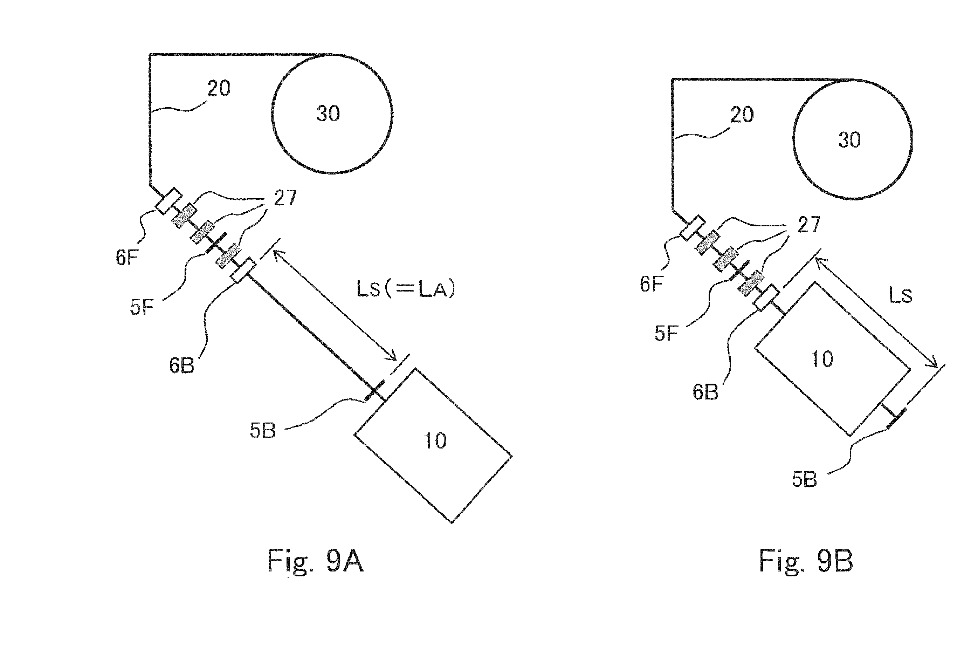

A particle therapy apparatus according to Embodiment 8 is the one to which technologies according to foregoing embodiments are applied and in which a single rotating gantry is disposed. FIGS. 9(a) and 9(b) are views for comparing the plant size of a conventional particle therapy apparatus with the plant size of a particle therapy apparatus according to Embodiment 8.

FIG. 9(a) and FIG. 9(b) illustrate the plant of a conventional example and the plant of the particle therapy apparatus according to Embodiment 8, respectively. In FIGS. 9(a) and 9(b), constituent elements that are the same as those in Embodiments 1 through 6 are designated by the same reference characters, and the detailed explanations therefor will be omitted.

As illustrated in FIGS. 9(a) and 9(b), in the respective particle therapy apparatuses according to a conventional example and Embodiment 8, the rotating gantry 10 is provided at the front of the transport system 20 communicating with the accelerator 30.

In the case where as in the past, it is required to secure the distance $L_S$ of the straight portion between the second steering electromagnet 6B and second position sensor 5B before the entrance of the rotating gantry 10, it is necessary to dispose the rotating gantry 10 in such a way that the distance between the branch point $P_B$ and the entrance of the rotating gantry 10 is longer than the distance $L_S$.

In contrast, in the case where as described in Embodiment 8, the straight portion between the two beam position sensor 5 is formed in such a way as to flank the entrance of the rotating gantry 10, the rotating gantry 10 can be disposed in such a way that the distance between the branch point $P_B$ and the entrance thereof is shorter than the distance $L_S$; thus, the area required for installing the plant can be reduced. This example has explained the arrangement of a rotating gantry and the peripheral devices thereof; however, it goes without saying that an irradiation chamber of another type may exist.

Description of Reference Numerals

1    entrance-side deflection electromagnet (deflection electromagnet)

1c     deflection path
1s     straight path
2      intermediate deflection electromagnet
3      exit-side deflection electromagnet
4      irradiation device
5      beam position sensor (trajectory correcting device)
5B     second position sensor
5F     first position sensor
6      steering electromagnet (trajectory correcting device)
6B     second steering electromagnet
6F     first steering electromagnet
7      irradiation chamber
8      vacuum window
9      duct
9V     vacuum duct
10     rotating gantry
19     containing unit
20     transport system
30     accelerator (radiation source)
B      particle beam
C      isocenter
$L_A$  distance of straight portion up to entrance of rotating gantry
$L_s$  distance between two beam sensors or distance between second steering electromagnet and second position sensor
$P_B$  branch point of beam transport path
$X_R$  rotation axis

## Claims

**1.** A particle therapy apparatus having a rotating gantry configured in such a way that an irradiation device rotates around a rotation axis and a particle beam is irradiated onto an irradiation subject,
wherein the rotating gantry is provided with a deflection electromagnet having a deflection path for radially deflecting a particle beam supplied along the rotation axis so as to guide the particle beam to the irradiation device and a straight path that is switchable with the deflection path and is for making the supplied particle beam travel in a straight manner; and wherein there is provided a trajectory correcting device having two position sensors that are arranged along the rotation axis so as to flank the deflection electromagnet.

**2.** The particle therapy apparatus according to claim 1,
wherein the trajectory correcting device has two pairs of steering electromagnets configured with steering electromagnets that are arranged at the upstream side of the deflection electromagnet and correct the trajectory position of a particle beam and steering electromagnets that correct the trajectory gradient of a particle beam.

**3.** The particle therapy apparatus according to claim 2,
wherein the two pairs of steering electromagnets and the two position sensors are arranged in such a way as to be on an extended line of the rotation axis.

**4.** The particle therapy apparatus according to any one of claims 2 and 3,
wherein the two pairs of steering electromagnets are arranged at the upstream side of the two position sensors.

**5.** The particle therapy apparatus according to any one of claims 2 and 3,
wherein the two pairs of steering electromagnets are arranged in such a way as to flank one, of the two position sensors, that is disposed at the more upstream side.

**6.** The particle therapy apparatus according to any one of claims 1 to 5,
wherein the other one, of the two position sensors, that is disposed at the more downstream side is provided inside an irradiation chamber for irradiating a particle beam onto the irradiation subject.

7.  The particle therapy apparatus according to claim 6,
    wherein a duct whose inside can be evacuated or filled with a predetermined gas atmosphere is provided in an attachable and detachable manner in the space between the deflection electromagnet and the other one, of the two position sensors, that is disposed at the more downstream side.

8.  The particle therapy apparatus according to claim 7,
    wherein a containing unit for containing the duct is provided on the rotating gantry.

Fig. 1

EP 2 959 944 A1

Fig. 2

EP 2 959 944 A1

Fig. 3

Fig. 4

EP 2 959 944 A1

Fig. 5

Fig. 6

EP 2 959 944 A1

Fig. 7A

Fig. 7B

EP 2 959 944 A1

Fig. 8A

Fig. 8B

Fig. 9A

Fig. 9B

EP 2 959 944 A1

<table>
<tr><td colspan="2">**INTERNATIONAL SEARCH REPORT**</td><td colspan="2">International application No.<br>PCT/JP2013/054568</td></tr>
</table>

**A. CLASSIFICATION OF SUBJECT MATTER**
*A61N5/10*(2006.01)i

According to International Patent Classification (IPC) or to both national classification and IPC

**B. FIELDS SEARCHED**

Minimum documentation searched (classification system followed by classification symbols)
A61N5/10

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2009-119123 A (Mitsubishi Electric Corp.), 04 June 2009 (04.06.2009), entire text; all drawings (Family: none) | 1-8 |
| A | JP 2008-264062 A (IHI Corp.), 06 November 2008 (06.11.2008), entire text; all drawings (Family: none) | 1-8 |
| A | JP 2003-282300 A (Hitachi, Ltd.), 03 October 2003 (03.10.2003), entire text; all drawings & US 2003/0183779 A1 & EP 1348465 A1 | 1-8 |

☒ Further documents are listed in the continuation of Box C.   ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 March, 2013 (12.03.13) | 26 March, 2013 (26.03.13) |

| Name and mailing address of the ISA/<br>Japanese Patent Office | Authorized officer |
|---|---|
| Facsimile No. | Telephone No. |

Form PCT/ISA/210 (second sheet) (July 2009)

<table>
<tr><td colspan="3">**INTERNATIONAL SEARCH REPORT**</td><td>International application No.<br>PCT/JP2013/054568</td></tr>
</table>

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2001-210498 A  (Mitsubishi Electric Corp.),<br>03 August 2001 (03.08.2001),<br>entire text; all drawings<br>(Family: none) | 1-8 |
| A | JP 11-204298 A  (Toshiba Corp.),<br>30 July 1999 (30.07.1999),<br>entire text; all drawings<br>(Family: none) | 1-8 |

Form PCT/ISA/210 (continuation of second sheet) (July 2009)

**EP 2 959 944 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003282300 A

- US 4917344 A